# EUROPÄISCHE PATENTSCHRIFT

(11) **EP 1 072 226 B1**
(45) Veröffentlichungstag und Bekanntmachung des Hinweises auf die Patenterteilung: **17.03.2004**
(21) Anmeldenummer: 00110573.3
(22) Anmeldetag: 18.05.2000
(51) Int. Cl.: A61B 17/22

(54) **Therapiegerät zur Stosswellenbehandlung eines Patienten**
Shock wave treatment therapy device
Appareil de traitement thérapeutique par ondes de choc

(30) Priorität: 29.07.1999 DE 19935724
(43) Veröffentlichungstag der Anmeldung: 31.01.2001
(60) Teilanmeldung: 02017280.5
(73) Patentinhaber: Richard Wolf GmbH, 75438 Knittlingen (DE)
(72) Erfinder: Bauer, Edgar, 76703 Kraichtal (DE); Sälzler, Stefan, 68753 Waghäusel (DE)
(74) Vertreter: Wilcken, Thomas, Dipl.-Ing.

(56) Entgegenhaltungen:
- EP-A- 0 106 292
- EP-A- 0 443 379
- DE-A- 3 926 380
- DE-A- 4 404 140
- DE-C- 19 630 180
- DE-U- 9 218 254
- US-A- 4 194 510
- US-A- 4 237 901
- US-A- 5 113 848
- US-A- 5 271 403
- US-A- 5 488 951

## Beschreibung

Die Erfindung geht aus von einem Therapiegerät zur Stoßwellenbehandlung eines Patienten, gemäß den Oberbegriffen der Patentansprüche 1 und 2.

Ein derartiges Therapiegerät zur Stoßwellenbehandlung, insbesondere ein Lithotriptor, ist in der DE-A-3926380 beschrieben. Das Gerät umfasst ein Hauptgehäuse mit einem über Energieleitungen betreibbaren Stoßwellengenerator und mit einem Koppelkissen, wobei zwischen dem Generator und dem distalen Wandbereich des Koppelkissens ein Aufnahmeraum für eine Koppelflüssigkeit gebildet ist, wobei die Menge der Koppelflüssigkeit in dem Aufnahmeraum zwecks Einstellung des Koppelkissens an dem Patienten steuerbar ist. Der die Koppelflüssigkeit enthaltende Aufnahmeraum kommuniziert mit einem ebenfalls mit Koppelflüssigkeit gefüllten Steuerraum, der wenigstens teilweise einen flexiblen Wandbereich aufweist, wobei dieser flexible Wandbereich mit einer gasförmigen Steuerkraft beaufschlagbar ist. Der Steuerraum ist als gesonderter Raum in einem starren, außen an dem Hauptgehäuse des Therapiegerätes befestigten Anschlussgehäuse vorgesehen, an dem wiederum eine Druckluftleitung geschlossen ist. Die Koppelflüssigkeit zirkuliert in einem geschlossenen Flüssigkeitskreislaufsystem, dessen Leitungen auch außerhalb des Therapiegerätes verlaufen und mit externen Einrichtungen kommunizieren, die insbesondere das Zirkulieren, Erwärmen und die Druckregelung der Koppelflüssigkeit bewirken.

Ein weiteres Therapiegerät ist in der EP-A-0 265 741 beschrieben. Es umfasst wenigstens einen Therapiekopf, der einen Stoßwellengenerator, eine patientenseitige Koppelmembran und einen dazwischen angeordneten Aufnahmeraum für Koppelflüssigkeit aufweist. An den Aufnahmeraum ist ein Flüssigkeitskreislauf angeschlossen, der neben einer Umwälzpumpe mehrere Ventile und eine Entgasungseinrichtung aufweist, um die den Aufnahmeraum gesteuert durchströmende Koppelflüssigkeit auch während der Behandlung blasenfrei zu halten. Um eine ausreichende Durchströmung des Therapiekopfes zu gewährleisten und um die Koppelmembran immer fest am Körper des zu behandelnden Patienten angedrückt halten zu können, ist es erforderlich, dass die Koppelflüssigkeit in dem Flüssigkeitskreislauf mit ausreichendem Druck und in ausreichender Menge umgewälzt wird, wozu die Leitungsquerschnitte des Kreislaufes relativ groß sein müssen. Dies führt zu dem weiteren Nachteil, dass die Handhabung des Therapiekopfes während der Patientenbehandlung wegen der relativ dicken Flüssigkeitsleitungen unhandlich und umständlich ist.

Ein anderes Therapiegerät ist in der DE-A-197 18 511 beschrieben. Es umfasst einen Therapiekopf, der über eine elektrische Leitung an eine Versorgungs- und Betriebseinheit angeschlossen ist. Der Therapiekopf ist eine geschlossene Baueinheit und enthält ein in seiner Menge nicht veränderbares Koppelmedium. Um größere Eindringtiefen des Therapiefokus in den Körper des Patienten zu ermöglichen, sind dem Therapiekopf entsprechende Koppelaufsätze zugeordnet. Koppelaufsätze sind Losteile und können leicht verloren gehen. Außerdem ist der Anwendungsbereich dieses Therapiekopfes durch die externen Koppelaufsätze oder Koppelkissen eingeschränkt, weil dessen Koppelmembran nur eine geringe Eindringtiefe des Therapiefokus des Therapiekopfes zulässt und weil die externen Koppelkissen keine stufenlose Einstellung des Therapiekopfes ermöglichen. Ferner ist die Anwendung des Therapiekopfes sehr umständlich, weil sich der Arzt mittels der Koppelkissen allmählich an den Therapieort herantasten muss.

In der DE-A-44 04 140 ist ein noch weiteres Therapiegerät beschrieben. Es dient der Behandlung von Schmerzzuständen und der Beeinflussung des vegetativen Nervensystems und umfasst einen Stoßwellengenerator, eine patientenseitige Koppelmembran, einen Aufnahmeraum mit Koppelflüssigkeit und eine zentrale Ortungseinrichtung. Der Therapiekopf des Stoßwellengenerators befindet sich vollkommen in der Koppelflüssigkeit des Aufnahmeraumes und muss daher rundherum gegenüber der Koppelflüssigkeit geschützt werden, wozu aufwendige Abdichtungen erforderlich sind. Des Weiteren ist eine aufwendige Verstellmechnik nötig, um den Therapiekopf auf den Therapiebereich einstellen zu können.

Die Aufgabe der Erfindung besteht darin, ein Therapiegerät der einleitend angeführten Art als Handgerät so zu verbessern, dass es unter Beibehaltung einer optimalen Behandlungsdurchführung eines Patienten während dieser Durchführung einfach und bequem gehandhabt werden kann.

Die Lösung dieser Aufgabe ist in den Patentansprüchen 1 und 2 angegeben.

Mit der erfindungsgemäßen Lösung ist die Handhabbarkeit des Therapiegerätes deutlich gesteigert und es steht bei kompakter Bauweise des Therapiegerätes eine relativ große Menge an Koppelflüssigkeit zur Verfügung. Des Weiteren kann der Strömungsquerschnitt der Steuerleitung für das Verringern und Vergrößern der Koppelflüssigkeitsmenge in dem Aufnahmeraum der Stoßwellengenerators sehr klein gehalten werden, weil es lediglich erforderlich ist, ein unter Druck stehendes Gasmedium, zum Beispiel Druckluft, durch diese Leitung zu führen, welches den Steuerraum des Therapiegerätes mit einer gasförmigen Druckkraft beaufschlagt: Durch die Druckkraft des Gasmediums wird die flexible Wand des.Steuerraumes entsprechend zusammengedrückt bzw. expandiert, so dass die in diesem Raum befindliche Koppelflüssigkeit in den Aufnahmeraum des Stoßwellengenerators gedrückt wird bzw. daraus wieder zurückströmen kann, um die Koppelmembran des Stoßwellengenerators therapiegerecht am Körper des Patienten anlegen zu können. Es brauchen also keine relativ träge strömenden Flüssigkeitsmengen mehr durch die Steuerleitung des gemeinsamen Versorgungsschlauches hindurch geleitet zu werden, sondern nur relativ schnell strömende Druckgase, wodurch die betreffende Druckgassteuerleitung einen wesentlich kleineren Durchmesser aufweist als eine entsprechende Flüssigkeitsleitung. Daraus folgt, dass der auch elektrische Leitungen für den Stoßwellengenerator enthaltende Versorgungsschlauch in seinem Außendurchmesser wesentlich kleiner gehalten werden kann als bisher, was die Handhabbarkeit des Therapiekopfes unter Beibehaltung einer genauer und großflächigen Positionierbarkeit der Koppelmembran des Stoßwellengenerators am Patienten erheblich steigert.

Eine vorteilhafte Ausgestaltung des erfindungsgemäßen Therapiegerätes besteht darin, dass der Versorgungsschlauch auch die Druckluftleitung für die Zuführung der Steuerkraft zur Beaufschlagung des flexiblen Wandbereiches des Steuerraumes enthält. Eine weitere vorteilhafte Ausgestaltung besteht darin, dass der Handgriff mit einer Bedienungstastatur für den Betrieb des Therapiegerätes versehen ist.

Die Erfindung ist nachstehend anhand eines in den anliegenden Zeichnungen dargestellten Ausführungsbeispieles näher erläutert. Es zeigen:
- Fig. 1: eine Versorgungseinrichtung, an der ein einziges Ausführungsbei spiel eines Therapiegerätes angeschlossen ist,
- Fig. 2: eine Versorgungseinrichtung, an der drei Einheiten des Ausführungsbeispiels angeschlossen sind,
- Fig. 3: das Ausführungsbeispiel in einem Axialschnitt,
- Fig. 4: eine schematische Schaltungsdarstellung eines an eine Versorgungseinrichtung angeschlossenen Ausführungsbeispiels,
- Fig.5: eine Fluidsteuerungseinrichtung für das Ausführungsbeispiel,
- Fig.6: ein weiteres abgeändertes Ausführungsbeispiel in Ansicht.

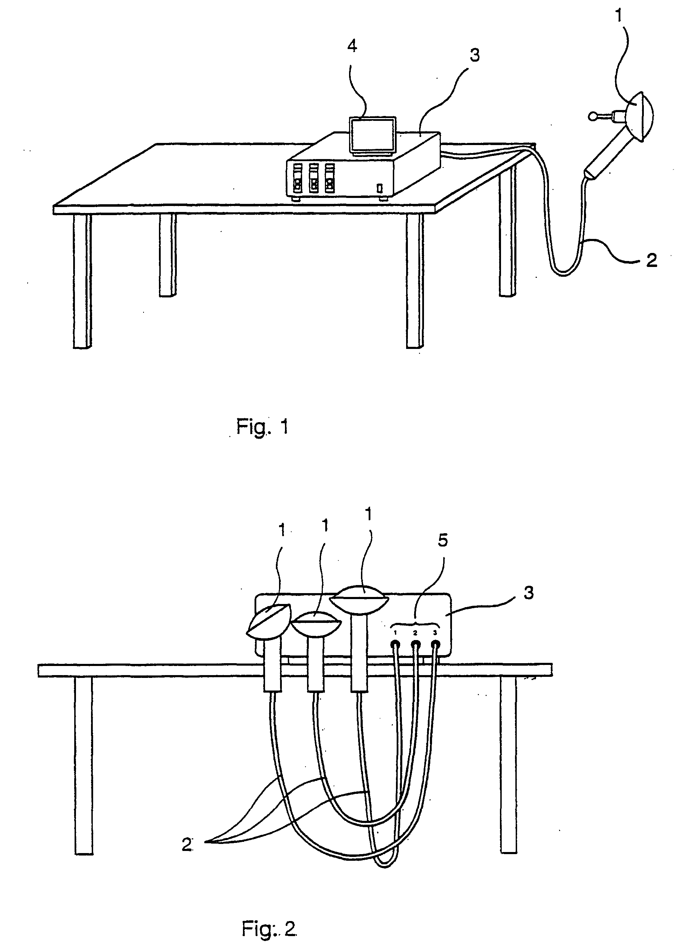

Fig. 1 zeigt ein einzelnes Therapiegerät 1 zur Stoßwellenbehandlung eines Patienten, das über einen Versorgungsschlauch 2 an eine Versorgungseinrichtung 3 mit einem Monitor 4 angeschlossen ist. Der Versorgungsschlauch 2 enthält mehrere Versorgungsleitungen für die elektrische und gasförmige Betätigung des Gerätes 1, dessen Betrieb von der Einrichtung 3 in an sich bekannter Weise gesteuert wird. Die Einrichtung 3 ist als Tischgerät ausgebildet; sie kann jedoch auch als Standgerät konstruiert sein.

Fig. 2 zeigt eine Versorgungseinrichtung 3 mit mehreren Therapiegeräten 1, zum Beispiel mit drei Therapiegeräten. Diese Einrichtung besitzt drei Steckplätze 5 für die Therapiegeräte, um für die jeweils gewünschte Behandlung das geeignete Therapiegerät auswählen zu können. Über eine Taste der Einrichtung 3 wird das gewünschte Therapiegerät betriebsbereit geschaltet. Die mehreren Therapiegeräte 1 nach Fig. 2 unterscheiden sich je nach dem erforderlichen Behandlungszweck. So können beispielsweise die Größe, die Apertur, die Eindringtiefe und die Leistung des Therapiegerätes unterschiedlich sein. Ferner können die Geräte 1 eine Ortungseinrichtung bekannter Art oder Positionierhilfen bekannter Art aufweisen. Ferner ist es möglich, daß die Ortungseinrichtungen und/oder Positionierhilfen zum Beispiel im Falle von Ultraschallsonden unterschiedliche Frequenzen aufweisen können. Ferner können die Therapiegeräte eine anatomisch oder der entsprechenden Indikation angepaßte Koppelmembran oder ein dementsprechend angepaßtes Therapiekopfgehäuse aufweisen. Selbstverständlich ist es auch möglich, daß die Versorgungsschläuche 2 eine unterschiedliche Länge haben können.

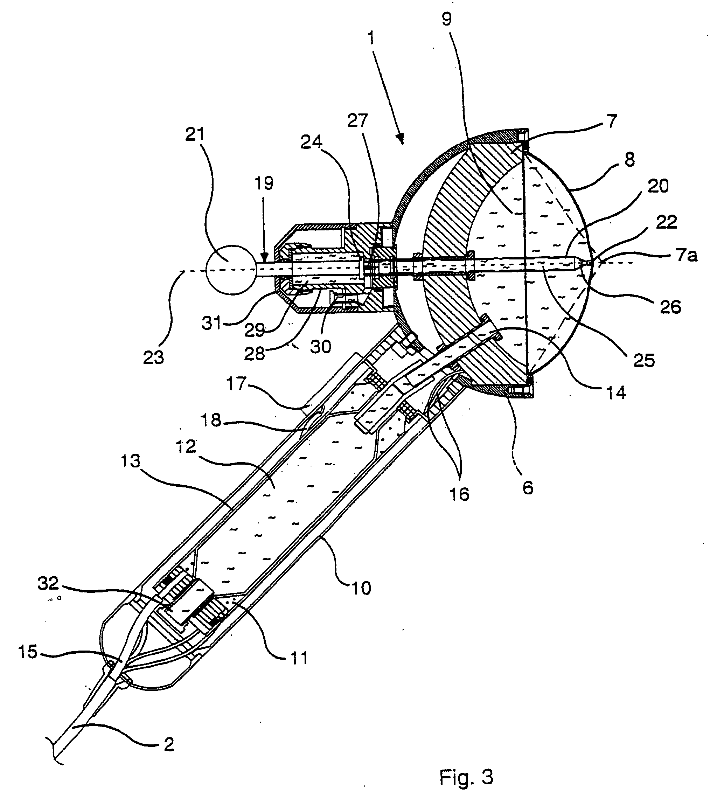

In Fig. 3 ist das Therapiegerät 1 im einzelnen dargestellt. In einem beispielsweise kalottenförmigen Hauptgehäuse 6 ist ein kalottenförmiger Stoßwellengenerator 7 angeordnet. An dem Hauptgehäuse ist eine den Stoßwellengenerator nach außen geschlossenwandig abschließende, kalottenförmige Koppelmembran 8 befestigt. Zwischen dem Generator 7 und der Koppelmembran 8 ist in bekannter Weise ein Raum 9 zur Aufnahme einer Koppelflüssigkeit gebildet. Die Menge der Koppelflüssigkeit in dem Aufnahmeraum 9 ist in Abhängigkeit von dem jeweiligen Therapievorgang veränderbar und wird mittels der Versorgungseinrichtung 3 über den Versorgungsschlauch 2 gesteuert, wie noch klar wird.

An das Hauptgehäuse 6 schließt sich ein starres Anschlußgehäuse 10 an, das gleichzeitig auch als ein länglicher, rohrförmiger Handgriff ausgebildet ist, mit dem das Therapiegerät 1 während der Behandlung gehalten oder an einem Stativ befestigt werden kann. Es ist möglich, wie in Fig. 3 gezeigt, in dem Anschlußgehäuse 10 bzw. in dem Handgriff einen weiteren Raum 11 mit starren Wänden vorzusehen. Dieser Raum bzw. alternativ das Anschlußgehäuse enthält einen Steuerraum 12, der wenigstens teilweise flexibel ausgebildet ist. Das Wandmaterial des Steuerraumes 12 ist ein fluiddichtes Material, wobei dieses Material zum Beispiel aus Gummi bestehen kann, so daß der Steuerraum, wie in Fig. 3 gezeigt, vollständig von einer flexiblen Wand 13 umgeben ist. Der somit flexible Steuerraum 12 ist des weiteren mit Koppelflüssigkeit gefüllt.

Über einen Kommunizierungsdurchlaß 14 steht der flexible Steuerraum 12 mit dem Aufnahmeraum 9 zwischen dem Stoßwellengenerator 7 und der Koppelmembran 8 in Strömungsverbindung.

An dem anderen Ende des Anschlußgehäuses 10 bzw. des Handgriffs ist der Versorgungsschlauch 2 angeschlossen, der zu der erwähnten Versorgungseinrichtung 3 führt. In diesem Versorgungsschlauch verläuft eine gasförmige Druckleitung, zum Beispiel eine Druckluftleitung 15, um die flexible Wand 13 des flexiblen Steuerraumes 12 in dem starren Raum 11 mit Druckluft beaufschlagen zu können. Im anwendungsbereiten Zustand sind der Aufnahmeraum 9 und der Steuerraum 12 einschließlich des Durchlasses 14 blasenfrei und vollständig mit Koppelflüssigkeit gefüllt, wobei die Koppelmembran 8 nicht unbedingt ihre äußerste Stellung aufweisen muß.

Wird nun die Koppelmembran 8 gegen die Behandlungsstelle des Patienten gedrückt bzw. daran angelegt, so strömt etwas Koppelflüssigkeit aus dem Raum 9 über den Durchlaß 14 zurück in den Steuerraum 12, dessen flexible Wand 13, oder dessen flexibler Wandbereich, sich dadurch ausdehnt. Über die Druckluftleitung 15 wird nun gesteuert Druckluft in den starren Raum 11 eingeleitet und damit Druck auf die Wand 13 des Steuerraumes 12 ausgeübt. Dadurch verkleinert sich das Volumen des Steuerraumes 12, wodurch Koppelflüssigkeit aus dem Steuerraum 12 über den Durchlaß 14 wieder in den Aufnahmeraum 9 gedrückt wird, um die Koppelmembran 8 großflächig und fest an den Körper des Patienten zu drücken. Die Druckluftleitung 15 hat im Vergleich zu einer bisher verwendeten Flüssigkeitsleitung einen wesentlich kleineren Strömungsquerschnitt, durch den hindurch jedoch Druckluft mit der erforderlichen Schnelligkeit geleitet werden kann, um die entsprechende Volumenänderung des Steuerraumes 12 vornehmen zu können. Durch eine solche, im Querschnitt relativ kleine Druckluftleitung wird erreicht, daß der Versorgungsschlauch, der auch die übrigen Leitungen für das Therapiegerät 1, beispielsweise die elektrischen Leitungen 16 für den Stoßwellengenerator 7, enthält, im Querschnitt wesentlich kleiner gehalten werden kann als bisher, wodurch die Handlichkeit des Therapiegerätes 1 wesentlich verbessert ist.

Zur weiteren verbesserten Handhabung und Bedienung des Therapiegerätes 1 kann das Anschlußgehäuse 10 bzw. der Handgriff mit einer Bedienungstastatur 17 versehen sein, die über eine elektrische Steuerleitung 18, die ebenfalls durch den Versorgungsschlauch 2 verläuft, mit der Versorgungseinrichtung 3 in Verbindung steht.

Das Therapiegerät 1 kann mit einer Positionierhilfe 19 für das richtige Anlegen des Gerätes am Körper des Patienten versehen sein. Gemäß dem Beispiel nach Fig. 3 besteht diese Positionierhilfe aus einer axial beweglichen Taststange 20, die entlang einer beliebig durch den Therapiefokus 7a des Stoßwellengenerators 7 verlaufenden Achse betätigbar ist. Hierzu besitzt die Taststange ein proximales Bedienungsteil 21, das außerhalb des Hauptgehäuses 6 vorgesehen ist. Das distale Ende der Taststange 20 ist als Tastspitze 22 aus röntgenundurchlässigem Material ausgebildet, beispielsweise in Form einer Metallkugel. Bevorzugterweise ist die Taststange 20 entlang der zentralen Symmetrieachse 23 des Stoßwellengenerators 7 axial bewegbar und mit einem Anschlagbund 24 versehen, um wenigstens die distale Endlage der Tastspitze 22 der Taststange 20 im Therapiefokus 7a des Stoßwellengenerators 7 bestimmen zu können. Die Tastspitze 22 kann also nur soweit vorgeschoben werden, bis ihre Position mit derjenigen des Therapiefokus des Stoßwellengenerators übereinstimmt. Die Symmetrieachse 23 des Stoßwellengenerators 7 ist exakt auf den Behandlungsort positioniert, wodurch die gewünschte Eindringtiefe definiert werden kann. Hierdurch kann der Behandlungsort am Patienten nach der Bio-Feedback-Behandlungsmethode ertastet werden.

In weiterer Fortbildung der Positionierhilfe 19 kann diese auch als Entlüftungseinrichtung beim Befüllen des Aufnahmeraumes 9 und des Steuerraumes 12 ausgebildet sein. Hierzu ist die Taststange 20 wenigstens teilweise als Hohlstange ausgebildet, und zwar vom Bereich ihrer Tastspitze 22 bis zu einer Stelle außerhalb des Hauptgehäuses 6. Entlang dieses Bereiches verläuft ein Entlüftungskanal 25, der im Bereich der Tastspitze 22 wenigstens eine Lufteinlaßöffnung 26 und an der Stelle außerhalb des Hauptgehäuses wenigstens eine Luftauslaßöffnung 27 aufweist. Diese Öffnung 27 kann unmittelbar abschließbar sein.

Die Luftauslaßöffnung 27 kann aber auch am äußeren Ende der Taststange 20 vorgesehen sein, z. B. wenn sich der Entlüftungskanal 25 durch die gesamte Taststange bis zu ihrem hinteren Ende hindurch erstreckt, an dem dann ein abschließbarer Anschlußstutzen vorgesehen ist.

Alternativ kann gemäß dem gezeichneten Beispiel auch so vorgegangen sein, daß die hinten aus dem Hauptgehäuse 6 herausragende Taststange 20 von einem sich an das Hauptgehäuse anschließenden Nebengehäuse 28 umgeben ist, wodurch ein Ringraum 29 gebildet ist, der in einen abschließbaren Auslaßstutzen 30 des Gehäuses 28 einmündet. Wenn es gewünscht wird, können das Nebengehäuse 28 und der

Auslaßstutzen 30 von einem Schutzgehäuse 31 umgeben sein. Die Auslaßöffnung 27 der Taststange 20 mündet also in den Ringraum 29, und von dort kann Koppelflüssigkeit während der Befüllung des Therapiegerätes 1 über den Auslaßstutzen 30 abströmen. Die Taststange 20 weist auch in diesem Fall einen solche Länge auf, daß sie aus den Gehäusen 28 und 31 hinten herausragt und mittels des Bedienungsteiles 21 betätigt werden kann. Die Bauteile 19 bis 31 gehören nicht zum Gegenstand der vorliegenden Erfindung.

Für die Befüllung des Aufnahmeraumes 9 und des Steuerraumes 12 mit Koppelflüssigkeit ist die Wandung 13 des Steuerraumes mit einem Einlaßstutzen 32 versehen. Zur Befüllung dieser beiden Räume mit Koppelflüssigkeit werden der Einlaßstutzen 32 und der Auslaßstutzen 30 an einen Befüllungskreislauf für Koppelflüssigkeit angeschlossen, der eine Entgasungseinrichtung aufweisen kann. Zur blasenfreien Befüllung des Aufnahmeraumes 9 ist die Taststange 20 in ihre distale Endstellung vorgeschoben. So ist die Koppelmembran 8 kegelförmig vorgespannt, und über die im vordersten Bereich befindliche Einlaßöffnung 26 der Taststange 20 wird bei entsprechender Haltung des Therapiegerätes 1 der Aufnahmeraum 9 vollkommen entlüftet und vollständig mit Koppelflüssigkeit gefüllt. Nach vollständiger, blasenfreier Befüllung der beiden Räume 9 und 12 werden die entsprechenden Stutzen 30 und 32 wieder verschlossen.

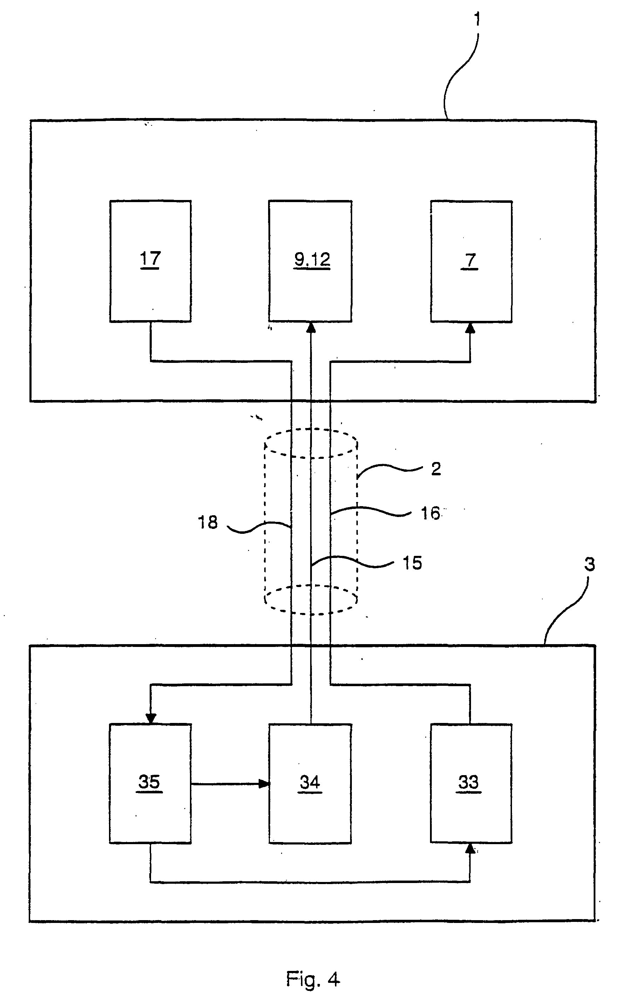

Fig. 4 zeigt eine schematische Schaltdarstellung zur allgemeinen Steuerung des Therapiegerätes 1. Man erkennt, daß der Stoßwellengenerator 7, die Räume 9 und 12 für die Koppelflüssigkeit und die Bedienungstastatur 17 des Therapiegerätes 1 über die in dem Versorgungsschlauch 2 verlaufenden Leitungen 16, 15 und 18 zu der Versorgungseinrichtung 3 verlaufen, wo die entsprechenden Steuereinheiten 33, 34 und 35 für die entsprechenden Teile des Therapiegerätes vorgesehen sind.

Die Steuerleitungen 15 und 16 verlaufen auch bei diesem Beispiel durch einen im Querschnitt kleinen Versorgungsschlauch 2, der sich unmittelbar an die Rückwand 6a des Hauptgehäuses 6 anschließen kann (nicht gezeigt). Sie können zunächst auch durch einen Handgriff (nicht dargestellt) verlaufen, der sich an die Rückwand 6a des Hauptgehäuses 6 anschließt, und dann in dem Schlauch 2 weitergeführt sein.

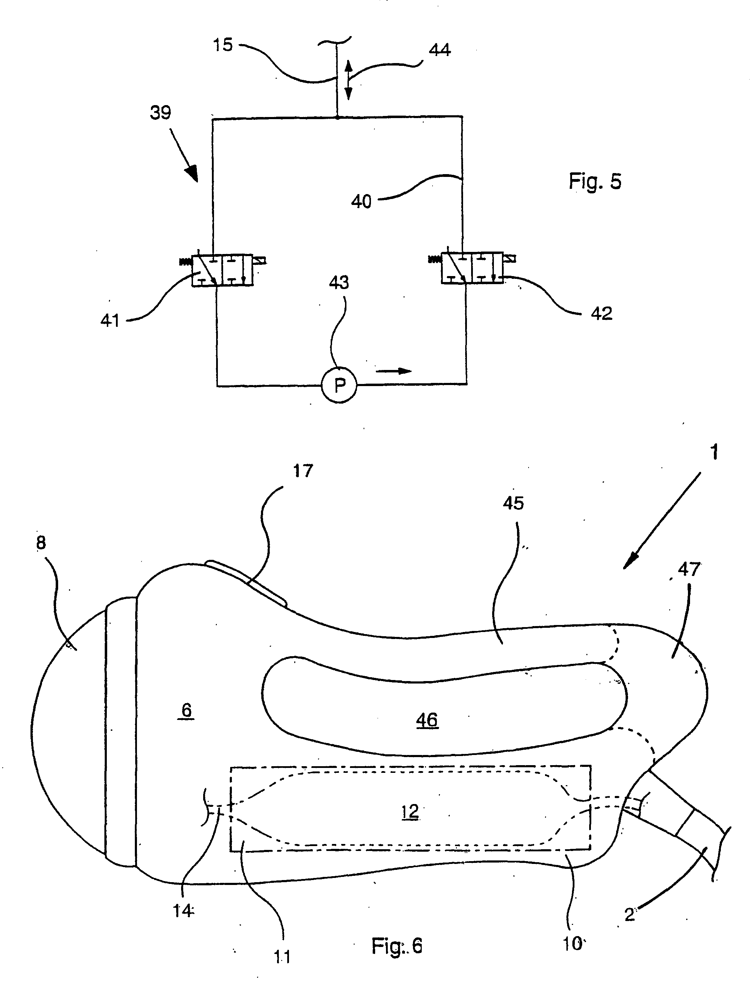

Fig. 5 zeigt eine pneumatische Steuerung 39 zur Erzeugung der Steuerkraft in der Druckluftleitung 15 zwecks Beaufschlagung der flexiblen Wandteile 13 des Steuerraumes 12. In einem Leitungskreis 40 sind zwei Steuerventile 41 und 42 sowie eine zwischen diesen beiden Ventilen angeordnete Umwälzpumpe 43 vorgesehen. Je nach Stellung der Steuerventile wird Druckluft gemäß dem Doppelpfeil 44 durch die Leitung 15 gepumpt, um den flexiblen Wandteil 13 des Steuerraumes 12 vor- oder zurückzubewegen. Über den Durchlaß 14 wird dadurch die entsprechende Menge an Koppelflüssigkeit in dem Aufnahmeraum 9 zwischen dem Stoßwellengenerator 7 und der Koppelmembran 8 je nach Erfordernis an dem zu behandelnden Patienten eingestellt.

Fig. 6 zeigt ein weiteres Ausführungsbeispiel. Das Hauptgehäuse 6 des Therapiegerätes 1 ist nach hinten verlängert ausgebildet, derart, daß die Verlängerung das Anschlußgehäuse 10, einen stegförmigen oder länglichen Handgriff 45 und einen Griffschlitz 46 dazwischen umfaßt. In Abänderung kann der proximale, teilweise gestrichelt angedeutete Bereich 47 des Gerätes 1 zwischen dem Gehäuse 10 und dem Handgriff 45 auch entfallen, so daß der Griffschlitz 46 hinten offen ist. In dem Anschlußgehäuse 10 ist der mit Druckluft aus dem Versorgungsschlauch 2 beaufschlagbare Raum 11 mit starren Wänden und darin der flexible Steuerraum 12 mit der Koppelflüssigkeit enthalten. Durch den Griffschlitz 46 greifen die Finger des Arztes, während er mit dem Daumen die in Nähe des Griffes 45 distal vorgesehene Tastatur 17 bedient. Diese Bauform des Therapiegerätes 1 hat den Vorteil, daß das Anschlußgehäuse 10 und.damit auch der darin befindliche Steuerraum 12 relativ groß ausgebildet sein können, so daß eine verhältnismäßig große Menge an steuerbarer Koppelflüssigkeit zur Verfügung steht, dennoch aber das Gerät 1 bequem gehalten und gehandhabt werden kann.

## Patentansprüche

1. Therapiegerät (1) zur Stoßwellenbehandlung eines Patienten, umfassend ein Hauptgehäuse (6) mit einem über einen Versorgungsschlauch (2) mit Energieleitungen betreibbaren Stoßwellengenerator (7) und einer Koppelmembran (8), wobei zwischen dem Generator und der Membran ein Aufnahmeraum (9) mit einer Koppelflüssigkeit gebildet ist, wobei die Menge der Koppelflüssigkeit in dem Aufnahmeraum zwecks Einstellung der Koppelmembran an dem Patienten steuerbar ist, wobei der Aufnahmeraum (9) mit einem ebenfalls mit Koppelflüssigkeit gefüllten Steuerraum (12), der wenigstens teilweise einen flexiblen, mit einer gasförmigen Steuerkraft beaufschlagbaren Wandbereich (13) aufweist, kommuniziert, und wobei der Steuerraum (12) als gesonderter Raum in einem starren, außen an dem Hauptgehäuse (6) befestigten , an eine Druckluftleitung (15) anschließbaren Anschlussgehäuse (10) vorgesehen ist, **dadurch gekennzeichnet, dass** ein an dem Hauptgehäuse (6) vorgesehener Handgriff rohrförmig ausgebildet ist und das Anschlussgehäuse (10) bildet oder enthält.

2. Therapiegerät (1) zur Stoßwellenbehandlung eines Patienten, umfassend ein Hauptgehäuse (6) mit einem über einen Versorgungsschlauch (2) mit Energieleitungen betreibbaren Stoßwellengenerator (7) und einer Koppelmembran (8), wobei zwischen dem Generator und der Membran ein Aufnahmeraum (9) mit einer Koppelflüssigkeit gebildet ist, wobei die Menge der Koppelflüssigkeit in dem Aufnahmeraum zwecks Einstellung der Koppelmembran an dem Patienten steuerbar ist, wobei der Aufnahmeraum (9) mit einem ebenfalls mit Koppelflüssigkeit gefüllten Steuerraum (12), der wenigstens teilweise einen flexiblen, mit einer gasförmigen Steuerkraft beaufschlagbaren Wandbereich (13) aufweist, kommuniziert, und wobei der Steuerraum (12) als gesonderter Raum in einem starren, außen an dem Hauptgehäuse (6) befestigten, an eine Druckluftleitung (15) anschließbaren Anschlussgehäuse (10) vorgesehen ist, **dadurch gekennzeichnet, dass** es eine hintere Verlängerung aufweist, die das Anschlussgehäuse (10), das länglich ausgebildet ist, mit dem Steuerraum (12), einen länglichen Handgriff (45) und einen Griffschlitz (46) dazwischen umfasst.

3. Therapiegerät nach Anspruch 1 oder 2, **dadurch gekennzeichnet, dass** der Versorgungsschlauch (2) die Druckluftleitung (15) für die Zuführung der Steuerkraft zur Beaufschlagung des flexiblen Wandbereiches (13) des Steuerraumes (12) enthält.

4. Therapiegerät nach Anspruch 1 oder 2, **dadurch gekennzeichnet, dass** der Handgriff mit einer Bedienungstastatur (17) für den Betrieb des Therapiegerätes (1) versehen ist.

## Claims

1. A therapy apparatus (1) for shock wave treatment of a patient, comprising a main housing (6) with a shock wave generator (7) able to be operated via a supply flexible tubing (2) with energy conduits, and with a coupling membrane (8), wherein between the generator and the membrane there is formed a receiving space (9) with a coupling fluid, wherein the quantity of the coupling fluid in the receiving space may be controlled for the purpose of adjusting the coupling membrane to the patient, wherein the receiving space (9) communicates with a control space (12) which is likewise filled with coupling fluid and which at least partly comprises a flexible wall region (13) able to be impinged with a gaseous control force, and wherein the control space (12) is provided as a separate space in a rigid connection housing (10) which is fastened on the outside to the main housing (6) and which is connectable to a pressurised air conduit (15), **characterised in that** a hand grip provided on the main housing (6) is designed tubular and forms or contains the connection housing (10).

2. A therapy apparatus (1) for shock wave treatment of a patient, comprising a main housing (6) with a shock wave generator (7) able to be operated via a supply flexible tubing (2) with energy conduits, and with a coupling membrane (8), wherein between the generator and the membrane there is formed a receiving space (9) with a coupling fluid, wherein the quantity of the coupling fluid in the receiving space may be controlled for the purpose of adjusting the coupling membrane to the patient, wherein the receiving space (9) communicates with a control space (12) which is likewise filled with coupling fluid and which at least partly comprises a flexible wall region (13) able to be impinged with a gaseous control force, and wherein the control space (12) is provided as a separate space in a rigid connection housing (10) which is fastened on the outside to the main housing (6) and which is connectable to a pressurised air conduit (15), **characterised in that** that it has a rear extension which comprises the connection housing (10) which is designed elongate, with the control space (12), an elongate hand grip (45) and a grip slot (46) therebetween.

3. A therapy apparatus according to claim 1 or 2, **characterised in that** the supply flexible tubing (2) contains the pressurised air conduit (15) for supplying the control force for impinging the flexible wall region (13) of the control space (12).

4. A therapy apparatus according to claim 1 or 2, **characterised in that** the hand grip is provided with an operating keyboard (17) for the operation of the therapy apparatus (1).

## Revendications

1. Appareil de traitement thérapeutique (1) pour le traitement d'un patient par ondes de choc, comprenant un boîtier principal (6) avec un générateur d'ondes de choc (7) pouvant fonctionner par l'intermédiaire d'un tuyau d'alimentation (2) avec des conducteurs d'énergie, et une membrane de couplage (8), une chambre de réception (9) avec un liquide de couplage étant formée entre le générateur et la membrane, la quantité de liquide de couplage dans la chambre de réception (9) étant susceptible d'être commandée à des fins de réglage de la membrane de couplage sur le patient, la chambre de réception (9) communiquant avec une chambre de commande (12) qui est également remplie d'un liquide de couplage et présente au moins en partie une zone de paroi flexible (13) pouvant être actionnée par une force de commande sous forme de gaz, et la chambre de commande (12) étant prévue sous forme de chambre séparée dans un boîtier de raccordement rigide (10) qui est fixé à l'extérieur sur le boîtier principal (6) et peut être raccordé à une conduite d'air comprimé (15), **caractérisé en ce qu'**une poignée prévue sur le boîtier principal (6) est d'une configuration tubulaire et forme ou renferme le boîtier de raccordement (10).

2. Appareil de traitement thérapeutique (1) pour le traitement d'un patient par ondes de choc, comprenant un boîtier principal (6) avec un générateur d'ondes de choc (7) pouvant fonctionner par l'intermédiaire d'un tuyau d'alimentation (2) avec des conducteurs d'énergie, et une membrane de couplage (8), une chambre de réception (9) avec un liquide de couplage étant formée entre le générateur et la membrane, la quantité de liquide de couplage dans la chambre de réception (9) étant susceptible d'être commandée à des fins de réglage de la membrane de couplage sur le patient, la chambre de réception (9) communiquant avec une chambre de commande (12) qui est également remplie d'un liquide de couplage et présente au moins en partie une zone de paroi flexible (13) pouvant être actionnée par une force de commande sous forme de gaz, et la chambre de commande (12) étant prévue sous forme de chambre séparée dans un boîtier de raccordement rigide (10) qui est fixé à l'extérieur sur le boîtier principal (6) et peut être raccordé à une conduite d'air comprimé (15), **caractérisé en ce qu'**il présente un prolongement arrière englobant le boîtier de raccordement (10), qui est d'une configuration allongée, et comporte la chambre de commande (12), une poignée (45) allongée, et, entre ces deux dernières, une fente de préhension (46).

3. Appareil de traitement thérapeutique selon la revendication 1 ou 2, **caractérisé en ce que** le tuyau d'alimentation (2) renferme la conduite d'air comprimé (15) pour l'amenée de la force de commande destinée à actionner la zone de paroi flexible (13) de la chambre de commande (12).

4. Appareil de traitement thérapeutique selon la revendication 1 ou 2, **caractérisé en ce que** la poignée est pourvue d'un clavier de commande (17) pour faire fonctionner l'appareil de traitement thérapeutique (1).
